(19) **Europäisches Patentamt / European Patent Office / Office européen des brevets**

(11) **EP 3 305 306 A1**

(12) # EUROPEAN PATENT APPLICATION

(43) Date of publication:
**11.04.2018 Bulletin 2018/15**

(51) Int Cl.:
***A61K 35/14*** *(2015.01)*

(21) Application number: **16002765.2**

(22) Date of filing: **23.12.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **04.10.2016 EP 16002124**

(71) Applicant: **Dr. Sarah Prinzessin von Isenburg 80333 München (DE)**

(72) Inventors:
• **NEUHANN-LORENZ, Constance 80333 München (DE)**
• **Von ISENBURG PRINZESSIN, Sarah 63633 Birstein (DE)**

(74) Representative: **Sattler de Sousa e Brito, Clara et al Arroba-IP Patentanwaltskanzlei Bahnhofstrasse 2 65307 Bad Schwalbach (DE)**

(54) **COMPOSITIONS COMPRISING ADJUSTABLE CONCENTRATIONS OF GROWTH FACTORS DERIVED FROM BLOOD SERUM AND CLOT HYPOXIA-CONDITIONED MEDIUM AND METHODS OF THEIR PRODUCTION**

(57) The present invention is based on the idea of extracorporeally simulating a wound in order to obtain wound healing/regenerative growth factor mixtures. The invention provides a method for separating the growth factors that are released from cells upon coagulation (coagulation-induced signaling phase) from the growth factors that are secreted by cells under hypoxia (hypoxia-induced signaling phase), then mixing the two phases at defined ratios to obtain a novel composition. Peripheral blood is obtained and allowed to clot or centrifuged to induce clotting. The serum is removed completely or partially and replaced with fresh medium of volume equal or less than the volume of serum removed. The clot is incubated in fresh medium at a temperature of 10 to 40°C, under hypoxia (1-10% $O_2$) for 1 to 7 days. The clot conditioned medium is then collected and mixed with the serum at a defined ratio to obtain the composition. Compositions comprising adjustable concentrations of growth factors derived from blood serum (coagulation-induced phase) and clot-conditioned medium (hypoxia-induced phase) can be added to suitable carriers, and can be topically applied or injected into a subject in order to aid wound healing, skin regeneration or rejuvenation.

EP 3 305 306 A1

**Description**

FIELD OF THE INVENTION

The present invention relates to compositions prepared from blood and blood serum. The compositions contain defined and adjustable concentrations of growth factors. The invention also concerns therapeutic and cosmetic methods in which these compositions are used. The invention further relates to methods and kits for producing these compositions.

BACKGROUND OF THE INVENTION

Blood coagulation is necessary directly after injury to prevent excessive bleeding. At this stage, initiation of new blood vessel formation (angiogenesis) would be logically counterproductive, since newly formed vessels are fragile and unstable. Hemostasis and angiogenesis are therefore two tightly controlled processes, which ensures that angiogenesis is only triggered once hemostasis has been completed. This is evident during wound healing, where angiogenesis only begins three days after wounding. This temporal regulation of the hemostatic and angiogenic phases is achieved through the spatiotemporally-controlled release of protein growth factors by cells into the wound microenvironment.

Following activation of coagulation, the fibrin clot entraps platelets at the site of injury, forming a hemostatic plug, which is gradually replaced by capillary-rich granulation tissue, and eventually collagen, leading to restoration of the original extracellular matrix (Reinke JM, Sorg H. Wound repair and regeneration. Eur Surg Res 2012; 49(1):35-43). Clot hypoxia potentiates the platelet-derived pro-angiogenic signaling generated through coagulation. Platelet activation and release of factors stored in their granules has been utilized as a strategy for obtaining angiogenic compositions based on platelet concentrates, such as platelet-rich plasma (PRP) and PRP gel/ platelet-rich fibrin matrix (PRFM). However, in addition to pro-angiogenic mediators, certain factors released by platelets (e.g. PF-4, TSP-1) are strongly anti-angiogenic. Such factors certainly play an important role in the aforementioned natural regulation of the hemostatic and angiogenic phases during wound healing. When present in excess (i.e. supraphysiological concentrations, such as those found in platelet concentrates), however, these factors might negatively impact the angiogenic effectiveness of platelet-rich products, by competing with or negating the effect of pro-angiogenic factors. This could explain the limited success of PRP products as chronic wound therapies to date.

Hypoxia is the primary angiogenic stimulus in physiological as well as pathological processes. Cell accumulation in the maturing wound bed or within a growing thrombus exacerbates the already compromised $O_2$ supply, by increasing local aerobic demand. Cell-mediated hypoxic conditioning of the hemostastic microenvironment results in an upregulated expression of a range of angiogenic growth factors such as VEGF, ANG, IL8, MMP-9 that are secreted by cells. This effect could be further potentiated though the simultaneous hypoxia-induced downregulation in the expression of anti-angiogenic factors, such as Thrombospondin 1 (TSP1).

*In vitro* hypoxic preconditioning of cells has been proposed to be a promising strategy for generating complex, yet physiological, angiogenic factor protein mixtures which can be delivered to ischaemic tissues (e.g. wounds, ulcers, burns) to aid re-perfusion, repair and regeneration. Among the various cell types that are suitable for hypoxic stress stimulation, peripheral blood cells (PBCs) represent an ideal autologous cell type, since their easy harvest and ample availability means that the need for lengthy cell population expansion cycles, required for example when using biopsy-harvested skin fibroblasts, is circumvented. PBCs can therefore be cultured directly after being obtained from the patient. Previous studies have shown that peripheral blood mononuclear cells (PBMCs) respond to stress (e.g. hypoxia/ischaemia, inflammation) by upregulating a wide range of angiogenic growth factors, such as VEGF, bFGF, IL-8, MMP-9, and have the ability to induce angiogenesis *in vitro* and *in vivo*. For example, it has been demonstrated that preconditioning PBMCs to hypoxia increases their survival and angiogenic potency upon implantation into ischaemic hindlimbs of mice. Also, intravenous administration of culture supernatant from irradiated apoptotic PBMCs confers cytoprotection to cardiomyocytes and inhibits tissue remodeling in rat and porcine acute myocardial infarction (AMI) models, while emulsions containing PBMC supernatant enhance wound closure. In various patient trials it was shown that transplantation of autologous mononuclear cells, from peripheral blood or bone marrow, increases leg perfusion in critical limb ischaemia, improves cardiac function after AMI, and accelerates the healing of refractory skin ulcers. These findings provide strong evidence that peripheral blood is a suitable source of angiogenic factor producing cells.

Conditioned culture media containing protein growth factors secreted by cells that are cultured under normoxia or hypoxia are well known in the art, and have been proposed as suitable compositions for inducing tissue repair and regeneration. Hypoxia conditioned plasma, i.e. plasma derived after extracorporeal conditioning of anticoagulated blood under physiological temperature (37°C) and physiological hypoxia (1-10% $O_2$), represents a special form of conditioned culture medium, in that its composition (concentrations and ratios of factors) is stoichiometrically (i.e. precisely) defined by the patient's peripheral blood cell type and number/count, in contrast to conditioned media typically obtained by *ex vivo/in vitro* reconstituted culture methods. This confers an important advantage when considering the inter-individual variation present in terms of gene expression and growth factor-induced cellular responses, and forms the basis for its utilization as an autologous therapy.

TECHNICAL PROBLEMS UNDERLYING THE
PRESENT INVENTION

As mentioned above, during wound healing the onset of hypoxia-induced angiogenesis is temporally distinct from the early inflammatory phase, in which platelets are primarily involved in hemostasis (angiogenesis is typically induced 3 to 4 days after wounding) (Reinke & Sorg, 2012, *supra*). While platelets might play a regulatory role, rather than a directly stimulatory role in new vessel formation, their contribution to the initiation, progression and completion of the wound healing process is self-evident. A key premise of this invention is that an optimal wound healing composition should comprise both the coagulation-induced, as well as the hypoxia-induced growth factor signalling phases. In nature, these two phases occur sequentially, i.e. the coagulation-mediated phase is induced immediately upon/soon after wounding through the release of growth factors by activated platelets, followed by the induction of the hypoxia-mediated phase which depends on growth factor production by blood cells and other cells present within or migrating into the wound. Previously described methods for producing conditioned culture media, including compositions comprising conditioned blood products, fail however to provide a way to segregate these two phases, as all the factors are released into the same (culture) medium (e.g. conditioned plasma or conditioned serum). A cumulative mixture is therefore obtained that contains all the required growth factors, but their concentrations and ratios can neither be controlled/adjusted, nor do they correlate with the concentrations/ratios at which these factors are progressively encountered with a natural wound.

This invention solves this problem by providing a method for first separating the two phases, in order to then be able to mix them at defined ratios in order to obtain tailor-made compositions that can eventually recapitulate the natural sequential expression of the two wound healing phases. The invention further solves this problem by providing a kit-of parts comprising the two phases in separate containers and a kit for preparing and separating the two phases.

SUMMARY OF THE INVENTION

In a first aspect the present invention relates to a kit-of-parts comprising:

> (a) a hypoxia-conditioned medium derived from blood; and
> (b) a serum

in separate containers.

In a second aspect the present invention relates to a solution of growth factors comprising a mixture of

> (a) a hypoxia-conditioned medium derived from blood; and
> (b) a serum.

In a third aspect the present invention relates to a method for preparing a kit-of parts, said method comprising the steps:

> (i) providing a blood sample from a subject;
> (ii) allowing the blood sample of step (i) to clot or inducing clotting in the blood sample of step (i), thereby obtaining a composition comprising a blood clot and serum;
> (iii) removing at least a part of the serum from the composition obtained in step (ii) and determining the volume of the serum removed;
> (iv) adding medium to the blood clot, wherein the volume of the added medium is equal to or less than the volume of the serum removed in step (iii);
> (v) incubating the clot in said medium at a temperature between 10°C and 40°C under hypoxic conditions for 1 to 7 days, thereby obtaining a hypoxia-conditioned medium;
> (vi) collecting at least a part of the hypoxia-conditioned medium obtained in step (v); and
> (vii) preparing a kit-of-parts comprising:
>
> > (a) the hypoxia-conditioned medium collected in step (vi) and
> > (b) at least a part of the serum removed in step (iii).

In a fourth aspect the present invention relates to a method for producing a solution of growth factors, said method comprising the steps:

> (i) providing a blood sample from a subject;
> (ii) allowing the blood sample of step (i) to clot or inducing clotting in the blood sample of step (i), thereby obtaining a composition comprising a blood clot and serum;
> (iii) removing at least a part of the serum from the composition obtained in step (ii) and determining the volume of the serum removed;
> (iv) adding medium to the blood clot, wherein the volume of the added medium is equal to or less than the volume of the serum removed in step (iii);
> (v) incubating the clot in said medium at a temperature between 10°C and 40°C under hypoxic conditions for 1 to 7 days, thereby obtaining a hypoxia-conditioned medium;
> (vi) collecting at least a part of the hypoxia-conditioned medium obtained in step (v);
> (vii) mixing the hypoxia-conditioned medium collected in step (vi) with at least a part of the serum removed in step (iii), thereby obtaining the solution of growth factors.

In a fifth aspect the present invention relates to a method for preparing a kit-of parts, said method comprising the steps:

(i) providing a first blood sample from a subject;
(ii) allowing the blood sample of step (i) to clot or inducing clotting in the blood sample of step (i), thereby obtaining a first composition comprising a blood clot and serum;
(iii) removing at least a part of the serum from the first composition obtained in step (ii) and determining the volume of the serum removed;
(iv) adding medium to the blood clot, wherein the volume of the added medium is equal to or less than the volume of the serum removed in step (iii);
(v) incubating the clot in said medium at a temperature between 10°C and 40°C under hypoxic conditions for 1 to 7 days, thereby obtaining a hypoxia-conditioned medium;
(vi) collecting at least a part of the hypoxia-conditioned medium obtained in step (v);
(vii) providing a second blood sample from a subject;
(viii) allowing the blood sample of step (vii) to clot or inducing clotting in the blood sample of step (vii), thereby obtaining a second composition comprising a blood clot and serum;
(ix) recovering at least a part of the serum from the second composition obtained in step (viii); and
(x) preparing a kit-of-parts comprising:

(a) the hypoxia-conditioned medium collected in step (vi) and
(b) the serum recovered in step (ix).

In a sixth aspect the present invention relates to a method for producing a solution of growth factors, said method comprising the steps:

(i) providing a first blood sample from a subject;
(ii) allowing the blood sample of step (i) to clot or inducing clotting in the blood sample of step (i), thereby obtaining a first composition comprising a blood clot and serum;
(iii) removing at least a part of the serum from the first composition obtained in step (ii) and determining the volume of the serum removed;
(iv) adding medium to the blood clot, wherein the volume of the added medium is equal to or less than the volume of the serum removed in step (iii);
(v) incubating the clot in said medium at a temperature between 10°C and 40°C under hypoxic conditions for 1 to 7 days, thereby obtaining a hypoxia-conditioned medium;
(vi) collecting at least a part of the hypoxia-conditioned medium obtained in step (v);
(vii) providing a second blood sample from a subject;
(viii) allowing the blood sample of step (vii) to clot or inducing clotting in the blood sample of step (vii), thereby obtaining a second composition comprising a blood clot and serum;
(ix) recovering at least a part of the serum from the second composition obtained in step (viii), thereby obtaining a serum; and
(x) mixing the hypoxia-conditioned medium collected in step (vi) with at least a part of the serum recovered in step (ix), thereby obtaining the solution of growth factors.

In a seventh aspect the present invention relates to a kit-of-parts prepared by the method of the third or fifth aspect.

In an eighth aspect the present invention relates to a solution of growth factors produced by the method of the fourth or sixth aspect.

In a ninth aspect the present invention relates to a composition comprising

(a) the solution of the second or of the eighth aspect; and
(b) a carrier.

In a tenth aspect the present invention relates to a solution of the second aspect or of the eighth aspect or to a composition of the ninth aspect for use in medicine.

In an eleventh aspect the present invention relates to a solution of the second aspect or of the eighth aspect or to a composition of the ninth aspect for use in the improvement of tissue blood perfusion, for use in the stimulation of angiogenesis, for use in the treatment of skin that has been debrided, for use in the treatment of excessive scarring, for use in the treatment of grafts, for use in the treatment of flaps, for use in the treatment of wounds, and/or for use in the treatment of tissue damage.

In a twelfth aspect the present invention relates to a cosmetic, non-therapeutic use of the solution of the second aspect or of the eighth aspect or of the composition of the ninth aspect.

In a thirteenth aspect the present invention relates to a kit for preparing serum and/or hypoxia-conditioned medium derived from blood, said kit comprising:

(a) a first syringe,
(b) a second syringe,
(c) optionally a third syringe,
(d) optionally a fourth syringe,
(e) a three-way valve,
(f) a filter,
(g) a cap,
(h) optionally a container containing sterile medium,
(i) optionally a catheter or butterfly cannula,
(j) optionally a sticking plaster,
(k) optionally a rack into which the first syringe can be placed and maintained in upright position, and
(l) optionally a spatula.

In a fourteenth aspect the present invention relates to a use of the kit of the thirteenth aspect in a method according to any one of the third, fourth, fifth, or sixth aspect.

This summary of the invention does not necessarily describe all features of the present invention. Other embodiments will become apparent from a review of the ensuing detailed description.

DESCRIPTION OF THE FIGURES

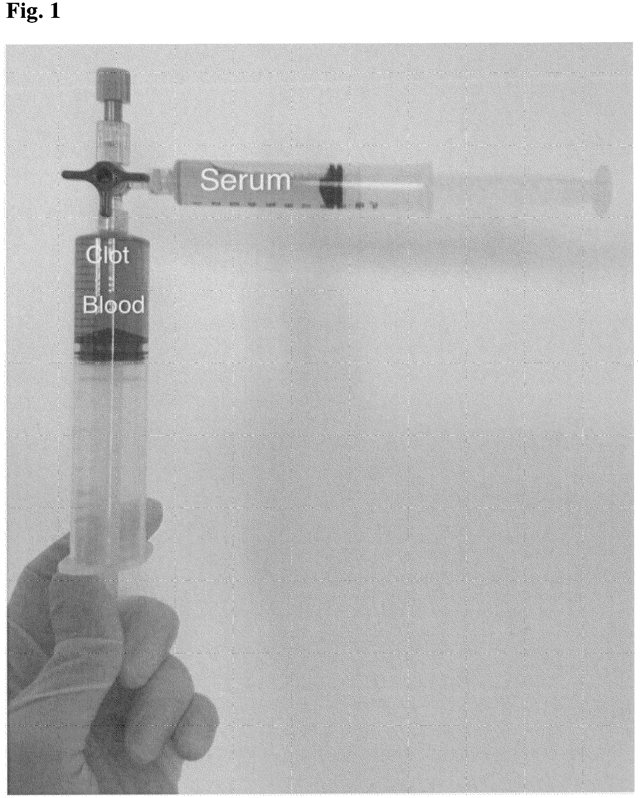

**Fig. 1:** Serum Separation. After a clot has formed in the first syringe (shown on the left side of the photograph), serum is withdrawn into the second syringe (shown at the top of the photograph).

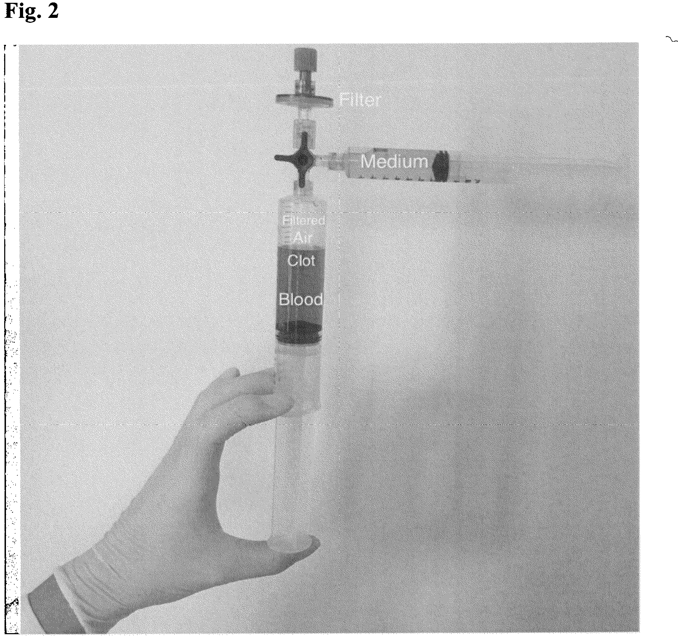

**Fig. 2:** Addition of air and medium. A filter was connected to the three way valve. Air was drawn into the first syringe through the filter. Then, the filter was covered with a cap. A third syringe filled with sterile medium was connected to the three-way valve. This is the situation shown in the photograph. Next, the three way valve will be turned so that the connection to the filter will be blocked and the first syringe and the third syringe will be connected. The medium will be pushed from the third syringe into the first syringe.

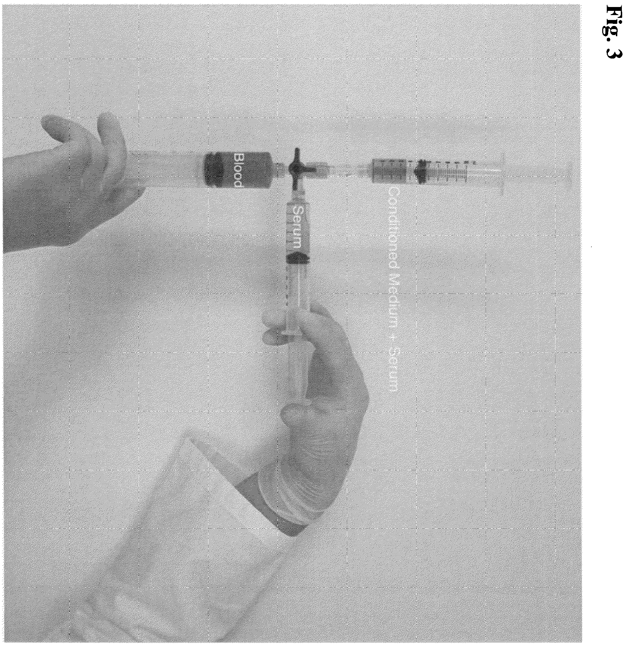

**Fig. 3:** Collection of new mixture (conditioned medium and serum). The filter was removed and a fourth empty syringe was attached to the three way valve. The second syringe containing the serum was reconnected to the three way valve. Conditioned medium from the first syringe was pushed into the fourth syringe. The three valve was turned so that the first syringe is blocked and the second syringe and the fourth syringe are connected. This is the situation shown in the photograph. Next, serum will be pushed into the fourth syringe, thereby producing a mixture of conditioned medium and serum.

DETAILED DESCRIPTION OF THE INVENTION

**Definitions**

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art to which this invention belongs.

Preferably, the terms used herein are defined as described in "A multilingual glossary of biotechnological terms: (IUPAC Recommendations)", Leuenberger, H.G.W, Nagel, B. and Kölbl, H. eds. (1995), Helvetica Chimica Acta, CH-4010 Basel, Switzerland).

Throughout this specification and the claims which follow, unless the context requires otherwise, the word "comprise", and variations such as "comprises" and "comprising", will be understood to imply the inclusion of a stated member, integer or step or group of members, integers or steps but not the exclusion of any other member, integer or step or group of members, integers or steps.

Several documents (for example: patents, patent applications, scientific publications, manufacturer's specifications, instructions etc.) are cited throughout the text of this specification. Nothing herein is to be construed as an admission that the invention is not entitled to antedate such disclosure by virtue of prior invention. Some of the documents cited herein are characterized as being *"incorporated by reference"*. In the event of a conflict between the definitions or teachings of such incorporated references and definitions or teachings recited in the present specification, the text of the present specification takes precedence.

As used herein, "hypoxic conditions" refer to oxygen concentrations of 10% or less.

As used herein, a "hypoxia-conditioned medium" refers to a solution obtainable by incubating a blood clot in a solution under hypoxic conditions for a certain amount of time and by subsequently removing the blot clot from the solution. Typically, the solution is a medium suitable for culturing blood cells. Typically, the incubation is carried out under hypoxic conditions at a temperature between 10°C and 40°C for 1 to 7 days. Under these conditions, the cells present in the blood clot secrete certain growth factors into the solution. Accordingly, the "hypoxia-conditioned medium" contains growth factors produced by the cells present in the blood clot. The expressions "hypoxia-conditioned medium", "clot-conditioned medium", and "clot hypoxia-conditioned medium" are used interchangeably herein.

As used herein, the term "serum" refers to a fraction of blood that is free from blood cells and clotting factors. Typically, serum can be obtained by centrifuging a coagulated blood sample.

**Embodiments of the Invention**

The present invention will now be further described. In the following passages different aspects of the invention are defined in more detail. Each aspect defined below may be combined with any other aspect or aspects unless clearly indicated to the contrary. In particular, any feature indicated as being preferred or advantageous may be combined with any other feature or features indicated as being preferred or advantageous.

In a first aspect the present invention is directed to a kit-of-parts comprising: (a) a hypoxia-conditioned medium derived from blood; and (b) a serum in separate containers.

In a second aspect the present invention is directed to a solution of growth factors comprising a mixture of (a) a hypoxia-conditioned medium derived from blood; and (b) a serum.

In some embodiments of the first aspect or the second aspect, the hypoxia-conditioned medium is derived from whole blood.

In some embodiments of the first aspect or the second aspect, the hypoxia-conditioned medium has a lower concentration of Platelet Factor 4 (PF4) than the serum. In preferred embodiments of the first aspect or the second aspect, the hypoxia-conditioned medium contains PF4 in a concentration between 0 ng/ml and 15,000 ng/ml, preferably between 5 ng/ml and 5,000 ng/ml.

In some embodiments of the first aspect or the second aspect, the hypoxia-conditioned medium contains at least one angiogenesis-related growth factor selected from the group consisting of VEGF, TSP1, IL8, Angiogenin (ANG), MMP-9, MMP-8, TIMP-1, and PDGF.

In some embodiments of the first aspect or the second aspect, the hypoxia-conditioned medium has a higher concentration of Vascular Endothelial Growth Factor (VEGF) than the serum. In preferred embodiments of the first aspect or the second aspect, the VEGF concentration in the hypoxia-conditioned medium is greater than 100 pg/ml; preferably in the range of 100 pg/ml to 500 ng/ml, more preferably in the range of 500 pg/ml to 50,000 pg/ml.

In some embodiments of the first aspect or the second aspect, the hypoxia-conditioned medium and/or the serum are derived from one or more blood samples obtained from the same subject.

In some other embodiments of the first aspect or the second aspect, the hypoxia-conditioned medium is derived from one or more blood samples obtained from a first subject and the serum is derived from one or more blood samples obtained from a second subject, wherein the first subject and the second subject are not the same subject.

In some embodiments of the first aspect or the second aspect, the hypoxia-conditioned medium and/or the serum is free of cells.

In some embodiments of the second aspect, the solution is free of cells.

In a third aspect the present invention is directed to a method for preparing a kit-of parts, said method comprising the steps:

(i) providing a blood sample from a subject;
(ii) allowing the blood sample of step (i) to clot or inducing clotting in the blood sample of step (i), thereby obtaining a composition comprising a blood clot and serum;
(iii) removing at least a part of the serum from the composition obtained in step (ii) and determining the volume of the serum removed;
(iv) adding medium to the blood clot, wherein the volume of the added medium is equal to or less than the volume of the serum removed in step (iii);
(v) incubating the clot in said medium at a temperature between 10°C and 40°C under hypoxic conditions for 1 to 7 days, thereby obtaining a hypoxia-conditioned medium;
(vi) collecting at least a part of the hypoxia-conditioned medium obtained in step (v); and
(vii) preparing a kit-of-parts comprising:

(a) the hypoxia-conditioned medium collected in step (vi) and
(b) at least a part of the serum removed in step (iii).

In a fourth aspect the present invention is directed to a method for producing a solution of growth factors, said method comprising the steps:

(i) providing a blood sample from a subject;
(ii) allowing the blood sample of step (i) to clot or inducing clotting in the blood sample of step (i), thereby obtaining a composition comprising a blood clot and serum;
(iii) removing at least a part of the serum from the composition obtained in step (ii) and determining the volume of the serum removed;
(iv) adding medium to the blood clot, wherein the volume of the added medium is equal to or less than the volume of the serum removed in step (iii);
(v) incubating the clot in said medium at a temperature between 10°C and 40°C under hypoxic conditions for 1 to 7 days, thereby obtaining a hypoxia-conditioned medium;
(vi) collecting at least a part of the hypoxia-conditioned medium obtained in step (v);
(vii) mixing the hypoxia-conditioned medium collected in step (vi) with at least a part of the serum removed in step (iii), thereby obtaining the solution of growth factors.

In a fifth aspect the present invention is directed to a method for preparing a kit-of parts, said method comprising the steps:

(i) providing a first blood sample from a subject;
(ii) allowing the blood sample of step (i) to clot or inducing clotting in the blood sample of step (i), thereby obtaining a first composition comprising a blood clot and serum;
(iii) removing at least a part of the serum from the first composition obtained in step (ii) and determining the volume of the serum removed;
(iv) adding medium to the blood clot, wherein the volume of the added medium is equal to or less than the volume of the serum removed in step (iii);
(v) incubating the clot in said medium at a temperature between 10°C and 40°C under hypoxic conditions for 1 to 7 days, thereby obtaining a hypoxia-conditioned medium;

(vi) collecting at least a part of the hypoxia-conditioned medium obtained in step (v);

(vii) providing a second blood sample from a subject;

(viii) allowing the blood sample of step (vii) to clot or inducing clotting in the blood sample of step (vii), thereby obtaining a second composition comprising a blood clot and serum;

(ix) recovering at least a part of the serum from the second composition obtained in step (viii); and

(x) preparing a kit-of-parts comprising:

(a) the hypoxia-conditioned medium collected in step (vi) and
(b) the serum recovered in step (ix).

In a sixth aspect the present invention is directed to a method for producing a solution of growth factors, said method comprising the steps:

(i) providing a first blood sample from a subject;

(ii) allowing the blood sample of step (i) to clot or inducing clotting in the blood sample of step (i), thereby obtaining a first composition comprising a blood clot and serum;

(iii) removing at least a part of the serum from the first composition obtained in step (ii) and determining the volume of the serum removed;

(iv) adding medium to the blood clot, wherein the volume of the added medium is equal to or less than the volume of the serum removed in step (iii);

(v) incubating the clot in said medium at a temperature between 10°C and 40°C under hypoxic conditions for 1 to 7 days, thereby obtaining a hypoxia-conditioned medium;

(vi) collecting at least a part of the hypoxia-conditioned medium obtained in step (v);

(vii) providing a second blood sample from a subject;

(viii) allowing the blood sample of step (vii) to clot or inducing clotting in the blood sample of step (vii), thereby obtaining a second composition comprising a blood clot and serum;

(ix) recovering at least a part of the serum from the second composition obtained in step (viii), thereby obtaining a serum; and

(x) mixing the hypoxia-conditioned medium collected in step (vi) with at least a part of the serum recovered in step (ix), thereby obtaining the solution of growth factors.

In some embodiments of the third aspect, the fourth aspect, the fifth aspect, or the sixth aspect, clotting is induced in method step (ii) by centrifuging the blood sample provided in step (i).

In some embodiments of the fifth aspect or the sixth aspect, clotting is induced in method step (viii) by centrifuging the blood sample provided in step (vii).

In some embodiments of the fifth aspect or the sixth aspect, the first blood sample and the second blood sample were obtained from the same subject.

In some other embodiments of the fifth aspect or the sixth aspect, the first blood sample was obtained from a first subject and the second blood sample was obtained from a second subject, wherein the first subject and the second subject are not the same subject.

While exposing PBCs to hypoxia is an effective method for inducing angiogenic factor upregulation, it faces the common problem that generally hinders the application of hypoxia-based therapies, namely the limited availability of $O_2$-controlled incubators/chambers in clinical settings. A promising approach to overcome this limitation is to allow PBCs to regulate their $O_2$ microenvironment, instead of exposing them to an artificial one, i.e. hypoxia produced within an incubator. It has been demonstrated that cell-mediated hypoxia can be achieved in the core of 3D collagen matrix depots, seeded at high density with dermal fibroblasts or vascular smooth muscle cells, by adjusting the total cell number and cell distribution within the depot (Cheema, U., Brown, R. A., Alp, B., and MacRobert, A. J., Spatially defined oxygen gradients and vascular endothelial growth factor expression in an engineered 3D cell model, Cellular and Molecular Life Sciences 65 (2008) 177-186, herein incorporated by reference in its entirety). In this work, a VEGF response was elicited by cells exposed to low levels of $O_2$ (~3% $O_2$), primarily within the construct core. Also, grafting cord blood mesenchymal stem cells as spheroids in ischaemic hind limbs of mice was shown to improve therapeutic efficacy due to enhanced cell survival and paracrine activity, effects mediated by hypoxic cell preconditioning within spheroid cultures (Bhang, S. H., Lee, S., Shin, J. Y., Lee, T. J., and Kim, B. S., Transplantation of cord blood mesenchymal stem cells as spheroids enhances vascularization, Tissue Eng Part A 18 (2012) 2138-2147, herein incorporated by reference in its entirety). In this study, culturing cells as monolayer, where cells were not exposed to hypoxia, abrogated these effects. These findings therefore indicate that cell-mediated hypoxia is a good alternative to externally-controlled (i.e. incubator) hypoxia. PBC pericellular O2 tension, being a function of cellular $O_2$ consumption, will predictably depend on the level of aerobic metabolism, as well as the population cell number/viability. Both these parameters are directly related to the PBC seeding density, which is initially determined by the ratio of blood volume to cross sectional surface area of the blood container. While lower ratios (e.g. <0.25 ml/cm$^2$) would guarantee uniform cell distribution/spread, and would therefore be more suitable to external (i.e. incubator-controlled) hypoxia, higher ratios (e.g. 0.25 - 50 ml/cm$^2$, preferably 0.25 - 5 ml/cm$^2$) would produce a higher cell density, therefore aiding the induction of persisting cell-mediated hypoxia.

Accordingly, in some embodiments of the third aspect, the fourth aspect, the fifth aspect, or the sixth aspect, the hypoxic conditions in the medium in step (v) are achieved through cell-mediated oxygen consumption within a normoxic incubator and/or within an oxygen-regulated incubator.

It is well established that cellular metabolic activity correlates with protein synthetic activity. Angiogenic factor expression by cultured PBCs will also depend on the ambient temperature, a direct controller of cellular metabolism. Culturing PBCs under physiological temperature (37°C) is therefore preferable, as this will promote higher growth factor production. Accordingly, in some embodiments of the third aspect, the fourth aspect, the fifth aspect, or the sixth aspect, the incubation in step (v) is carried out at a temperature between 20°C to 40°C, preferably between 25°C and 40°C, more preferably between 30°C and 40°C, even more preferably between 35°C and 40°, and most preferably at about 37°C.

In some embodiments of the third aspect, the fourth aspect, the fifth aspect, or the sixth aspect, the incubation in step (v) is carried out at an oxygen concentration of between 1 and 10%, preferably between 1% and 5%.

In some embodiments of the third aspect, the fourth aspect, the fifth aspect, or the sixth aspect, the volume of the medium added in step (iv) is between 1/200 and 2/3 of the volume of the serum removed in step (iii), for example 1/200, 1/100, 1/20, 1/10, 1/5, 1/2 or 2/3.

In some embodiments of the third aspect, the fourth aspect, the fifth aspect, or the sixth aspect, step (vi) comprises the repeated collection of hypoxia-conditioned medium by carrying out the following steps:

(1) collecting at least a part of the hypoxia-conditioned medium at a first time point;
(2) adding medium to the blot clot, thereby replacing the hypoxia-conditioned medium collected in step (1),
(3) continuing incubation,
(4) collecting at least a part of the hypoxia-conditioned medium at a second time point, and
(5) optionally repeating steps (2) to (4) between 1 time and 5 times.

In preferred embodiments of the third aspect, the fourth aspect, the fifth aspect, or the sixth aspect, the volume of the medium added in step (iv) and/or the medium added in step (2) is equal to or less than the volume of the serum removed in step (iii).

In further preferred embodiments of the third aspect, the fourth aspect, the fifth aspect, or the sixth aspect, the total volume of the medium added in step (iv) and the medium added in step (2) is equal to or less than the volume of the serum removed in step (iii).

In further preferred embodiments of the third aspect, the fourth aspect, the fifth aspect, or the sixth aspect, the solution of growth factors obtained in step (vii) of the fourth aspect or in step (x) of the sixth aspect is obtained by mixing a given volume of hypoxia-conditioned medium (*Vhcm*) with a volume of serum (*Vs*) that fulfils the following inequality:

$$V_s \leq \frac{V_{hcm} * V_{s_{total}}}{V_{hcm_{total}}} - V_{hcm}$$

wherein *Vs* is the volume of serum that can be used for mixing,
wherein $V_{hcm}$ is the volume of hypoxia-conditioned medium used for mixing,
wherein $V_s$ is the total volume of the serum removed in step (iii),
wherein $V_{hcm_{total}}$ is the total volume of the medium added in step (iv), and
wherein additionally the following condition applies:
$V_{hcm_{total}} \leq V_{s_{total}}$.

In some embodiments of the third aspect, the fourth aspect, the fifth aspect, or the sixth aspect, the medium is selected from the group consisting of serum-containing culture medium, serum-free culture medium, a solution that is isotonic with blood, a culture medium with glucose, a culture medium without glucose, a culture medium with albumin and a culture medium without albumin.

In a seventh aspect the present invention is directed to a kit-of-parts prepared by the method of the third aspect or the fifth aspect.

In an eighth aspect the present invention is directed to a solution of growth factors produced by the method of the fourth or the sixth aspect.

In a ninth aspect the present invention is directed to a composition comprising (a) the solution of the second aspect or the solution of the eighth aspect; and (b) an excipient.

In some embodiments of the ninth aspect, the composition is a pharmaceutical composition. In preferred embodiments, the excipient is a pharmaceutically acceptable excipient.

In some embodiments of the ninth aspect, the composition is a cosmetic composition. In preferred embodiments, the excipient is a cosmetically acceptable excipient.

In some embodiments of the ninth aspect, the excipient is fibrinogen. In some embodiments, the fibrinogen is exogenous fibrinogen, i.e. fibrinogen obtained from a different subject than the subject to which the composition is applied or administered.

In some embodiments of the ninth aspect, the composition further comprises: (c) a carrier. Preferably, the carrier is a pharmaceutically acceptable carrier or a cosmetically acceptable carrier. In preferred embodiments, the carrier is in the form of a cream, an emollient, an ointment, a gel, a sol-gel, a spray, a powder, a mesh, a sponge, a patch, a dressing, nanoparticles, microparticles, nanofibers or microfibers. In further preferred embodiments, the carrier comprises one or more substances selected from the group consisting of proteins (e.g. collagen, elastin, fibrin, or fibronectin), polysaccharides (e.g. alginate, chitin, or cellulose), glycosaminoglycan (e.g. hyaluronic acid, dermatan sulphate, keratin sulphate, chondroitin sulphate, heparan sulphate, or heparin sulphate), and synthetic polymers (e.g. polyglycolic acid, polylactic acid, polycaprolactone, or polylactic co-glycolic acid).

Without wishing to be bound by any particular theory, the inventors believe that using fibrinogen as excipient or using particular carriers provides the following advantages: Spatial factor gradients promote chemotactic migration of cells, including endothelial cells towards a target site, and are important for stimulation of directional angiogenesis. To achieve such gradients, growth factors could be loaded onto suitable polymeric carriers that can be topically applied to a wound or be locally injected intradermally/subcutaneously through gel or sol-gel vehicles. Gradual factor release from the vehicle establishes a spatial gradient. In addition to using exogenous gels and sol-gels, localized delivery could be achieved through the addition of exogenous fibrinogen (autologous or of allogeneic/xenogenic origin) to a composition, and combining this solution with thrombin/calcium in order to induce the formation of a fibrin gel-matrix similar to the one that forms upon coagulation. The *in vivo*-formed fibrin matrix then sequesters the factors at the site of administration, by specific binding (e.g. VEGF) and/or passive trapping, and ensures their controlled release as it occurs within a wound. This also helps avoiding unwanted side-effects such as vascular leakage and ectopic angiogenesis. A further functionality of such factor-loaded matrices is that they can serve as scaffolds for migrating host cells (e.g. fibroblasts, endothelial cells) at a defect site, hence promoting tissue repair and regeneration.

In a tenth aspect the present invention is directed to a solution of the second aspect or a solution of the eighth aspect or a composition of the ninth aspect for use in medicine.

In an eleventh aspect the present invention is directed to a solution of the second aspect or a solution of the eighth aspect or a composition of the ninth aspect for use in the improvement of tissue blood perfusion, for use in the stimulation of angiogenesis, for use in the treatment of skin that has been debrided (e.g. by mechanical, chemical, or laser debridement, microdermabrasion, or needling), for use in the treatment of excessive scarring (including treatment of acne, keloids and hypertrophic scars), for use in the treatment of grafts (e.g. skin grafts or fat grafts), for use in the treatment of flaps (e.g. local flaps or free flaps), for use in the treatment of wounds (e.g. diabetic ulcers, arterial ulcers, venous-stasis wounds, decubital ulcers, radiation-induced wounds, steroid-induced wounds, ischaemic wounds, post-wounding and post-grafting ischaemic tissue, incisions, lacerations, abrasions or burns), and/or for use in the treatment of tissue damage (e.g. tissue damage caused by disorders selected from the group consisting of diabetes; peripheral artery disease; vascular occlusive disease; atherosclerosis; myointimal hyperplasia; thromboangiitis obliterans; thrombotic disorders; mesenteric ischemia; limb ischemia; peripheral artery stenosis; vasculitis; infarctions including cerebral and myocardial infarctions; traumatic injuries; bone fractures; osteoporosis; arthritis (including osteoarthritis); rheumatism; cartilage rupture or detachment; torn or ruptured tendons; spinal injuries; muscular dystrophies; amyotrophic lateral sclerosis; cancer; AIDS; central nerve injury; peripheral nerve injury; central nerve degeneration; and peripheral nerve degeneration).

In an alternative wording, the eleventh aspect of the present invention is directed to the use of a solution of the second aspect or a solution of the eighth aspect or a composition of the ninth aspect in the preparation of a pharmaceutical composition for the improvement of tissue blood perfusion, for the stimulation of angiogenesis, for the treatment of skin that has been debrided (e.g. by mechanical, chemical, or laser debridement, microdermabrasion, or needling), for the treatment of excessive scarring (including treatment of acne, keloids and hypertrophic scars), for the treatment of grafts (e.g. skin grafts or fat grafts), for the treatment of flaps (e.g. local flaps or free flaps), for the treatment of wounds (e.g. diabetic ulcers, arterial ulcers, venous-stasis wounds, decubital ulcers, radiation-induced wounds, steroid-induced wounds, ischaemic wounds, post-wounding and post-grafting ischaemic tissue, incisions, lacerations, abrasions or burns), and/or for the treatment of tissue damage (e.g. tissue damage caused by disorders selected from the group consisting of diabetes; peripheral artery disease; vascular occlusive disease; atherosclerosis; myointimal hyperplasia; thromboangiitis obliterans; thrombotic disorders; mesenteric ischemia; limb ischemia; peripheral artery stenosis; vasculitis; infarctions including cerebral and myocardial infarctions; traumatic injuries; bone fractures; osteoporosis; arthritis (including osteoarthritis); rheumatism; cartilage rupture or detachment; torn or ruptured tendons; spinal injuries; muscular dystrophies; amyotrophic lateral sclerosis; cancer; AIDS; central nerve injury; peripheral nerve injury; central nerve degeneration; and peripheral nerve degeneration).

In another alternative wording, the eleventh aspect of the present invention is directed to a method for the improvement of tissue blood perfusion, for the stimulation of angiogenesis, for the treatment of skin that has been debrided (e.g. by mechanical, chemical, or laser debridement, microdermabrasion, or needling), for the treatment of excessive scarring (including treatment of acne, keloids and hypertrophic scars), for the treatment of grafts (e.g. skin grafts or fat grafts), for the treatment of flaps (e.g. local flaps or free flaps), for the treatment of wounds (e.g. diabetic ulcers, arterial ulcers, venous-stasis wounds, decubital ulcers, radiation-induced wounds, steroid-induced wounds, ischaemic wounds, post-wounding and post-grafting ischaemic tissue, incisions, lacerations, abrasions or burns), and/or for the treatment of tissue damage (e.g. tissue damage caused by disorders selected from the group consisting of diabetes; peripheral artery disease; vascular occlusive disease; atherosclerosis; myointimal hyperplasia; thromboangiitis obliterans; thrombotic disorders; mesenteric ischemia; limb ischemia; peripheral artery stenosis; vasculitis; infarctions including cerebral and myocardial infarctions; traumatic injuries; bone fractures; osteoporosis; arthritis (including osteoarthritis); rheumatism; cartilage rupture or detachment; torn or ruptured tendons; spinal injuries; muscular dystrophies; amyotrophic lateral sclerosis; cancer; AIDS; central nerve injury; peripheral nerve injury; central nerve degeneration; and peripheral nerve degeneration), said method comprising the step of administering a therapeutic amount of a solution of the second aspect or a solution of the eighth aspect or a composition of the ninth aspect to a subject in need thereof.

In some embodiments of the eleventh aspect, the solution or the composition is for autologous administration.

In some embodiments of the eleventh aspect, the solution or the composition is for allogeneic application.

In some embodiments of the eleventh aspect, the solution or the composition is for epidermal administration.

In some embodiments of the eleventh aspect, the solution or the composition is for intradermal administration.

In some embodiments of the eleventh aspect, the solution or the composition is for subcutaneous administration.

In a twelfth aspect the present invention is directed to a cosmetic, non-therapeutic use of the solution of the second aspect or the solution of the eighth aspect or of the composition of the ninth aspect.

In some embodiments of the twelfth aspect, the use is for improving the appearance of skin (e.g. skin that has been altered through chronological ageing or photodamage), for stimulating new hair growth, for augmenting soft tissue, for reducing wrinkles, for skin rejuvenation, for improving the appearance of scars, for reducing excessive skin pigmentation, and/or for improving pigmentation in skin with reduced pigmentation.

In an alternative wording, the twelfth aspect of the present invention is directed to a cosmetic, non-therapeutic method for improving the appearance of skin (e.g. skin that has been altered through chronological ageing or photodamage), for stimulating new hair growth, for augmenting soft tissue, for reducing wrinkles, for skin rejuvenation, for improving the appearance of scars, for reducing excessive skin pigmentation, and/or for improving pigmentation in skin with reduced pigmentation, said method comprising the step of administering an effective amount of a solution of the second aspect or a solution of the eighth aspect or a composition of the ninth aspect to a subject in need of such cosmetic treatment.

In a thirteenth aspect the present invention is directed to a kit for preparing serum and/or hypoxia-conditioned medium derived from blood, said kit comprising:

(a) a first syringe,
(b) a second syringe,
(c) optionally a third syringe,
(d) optionally a fourth syringe,
(e) a three-way valve,
(f) a filter, and
(g) a cap.

In preferred embodiments of the thirteenth aspect, the first syringe has a volume of about 30 ml. In further preferred embodiments of the thirteenth aspect, the first syringe is certified for incubation at 37°C for 1 to 7 days. In further preferred embodiments of the thirteenth aspect, the first syringe is labelled with the word 'UP' (or 'TOP' or a similar expression) at the end with the opening and labelled with the word 'DOWN' (or 'BOTTOM' or a similar expression) at the end with the plunger.

In preferred embodiments of the thirteenth aspect, the second syringe has a volume of about 10 ml.

In preferred embodiments of the thirteenth aspect, the third syringe has a volume of about 10 ml.

In preferred embodiments of the thirteenth aspect, the fourth syringe has a volume of about 10 ml.

In preferred embodiments of the thirteenth aspect, the first, the second and (if present) the third and fourth syringes are removably connectable to the three-way valve, e.g. by Luer lock connections.

In preferred embodiments of the thirteenth aspect, the kit comprises at least one filter (e.g. 1 filter, 2 filters, 3 filters, 4 filters, 5 filters, 6 filters, 7 filters, 8 filters, 9 filters, or 10 filters). In particularly preferred embodiments, the kit comprises at least two filters. In more preferred embodiments, the kit comprises at least three filters. In even more preferred embodiments, the kit comprises at least four filters. In most preferred embodiments, the kit comprises at least five filters. In preferred embodiments of the thirteenth aspect, the filter is a 0.2 μm filter or the filters are 0.2 μm filters. In further preferred embodiments of the thirteenth aspect, the filter is / the filters are removably connectable to the three-way valve and/or to any of the four syringes, e.g. by Luer lock connections.

In preferred embodiments of the thirteenth aspect, the cap is removably connectable to the three-way valve and/or to any one of the four syringes and/or to any one of the filters, e.g. by Luer lock connections.

In some embodiments of the thirteenth aspect, the kit further comprises a container containing sterile medium. In preferred embodiments, the sterile medium is selected from the group consisting of serum-containing culture medium, serum-free culture medium, a solution that is isotonic with blood, a culture medium with glucose, a culture medium without glucose, a culture medium with albumin and a culture medium without albumin.

In some embodiments of the thirteenth aspect, the kit further comprises a catheter (e.g. Vasuflo-Int) or a butterfly cannula.

In some embodiments of the thirteenth aspect, the kit further comprises a sticking plaster (i.e. an adhesive bandage in American English), e.g. Band-Aid™ or Elastoplast™.

In some embodiments of the thirteenth aspect, the kit further comprises a rack into which the first syringe can be placed and maintained in upright position.

In some embodiments of the thirteenth aspect, the kit further comprises a spatula. The spatula may be used to mix serum or hypoxia-conditioned medium prepared by using the kit with skincare products.

In a fourteenth aspect the present invention is directed to a use of the thirteenth aspect in a method according to any one of the third, fourth, fifth, or sixth aspect.

BEST MODE FOR CARRYING OUT THE INVENTION

The solution of the invention can be produced by a method comprising the steps of obtaining a blood sample, preferably from a peripheral vein, and allowing the blood to clot or centrifuging the blood to induce clotting, and removing the serum completely or partially, preferably without disturbing the clot, then replacing the serum with fresh medium , and incubating the clot in fresh medium at a temperature of 10 to 40°C (preferably at 37°C) under hypoxia (1-10% $O_2$, preferably 1-5% $O_2$) for 1 to 7 days (preferably 1-4 days), and collecting the conditioned medium and mixing it with the serum at a defined ratio to obtain a solution of the invention.

The fresh medium used for clot incubation can be a suitable cell culture medium such as serum-containing or serum-free culture medium, a solution that is isotonic with blood, a culture medium with or without glucose, a culture medium with or without albumin. The serum, clot conditioned medium and/or the mixed solution can be filtered to remove cells or cellular material. During the incubation period (i.e. 1 to 7 days) the serum can be frozen (-10°C to -80°C) and stored, then thawed for mixing. The mixed composition can also be frozen (-10°C to -80°C) for up to 12 months while maintaining (at least part of) its bioactivity. The mixed composition can be also lyophilized or desiccated and reconstituted in a buffer.

This method allows for precise control of the concentration of protein growth factors in clot conditioned medium by controlling the volume of fresh medium added after serum removal. Preferably, the serum is replaced with a fresh medium of volume equal to or less than the volume of the serum obtained/removed in order to increase the concentration of the factors secreted by clotted blood cells into the conditioned medium above the concentration that would be achieved if the factors were secreted directly into the original serum volume during conditioning. In this way the factors can be concentrated by 1.5 fold, 2 fold, 5 fold, 10 fold, 20 fold, 100 fold, 200 fold or more. For example VEGF, which has an average concentration in serum of 60-700 pg/ml could be concentrated up to 500 ng/ml. This large increase in factor concentration is significantly higher than the increase that can be achieved by conditioning blood cells under hypoxia. The concentration of factors released in the serum can be indirectly adjusted by mixing it with clot conditioned medium. Serum will contain a significantly higher concentration of platelet-derived factors, such as anti-angiogenic factor PF4 that is normally present in serum at ca. 3000 - 15000 ng/ml, than clot conditioned medium. The preferred mixing volume ratio of clot conditioned medium to serum is such that the concentration of factors in the clot conditioned medium is never diluted, as a result of mixing, below what it would have been if the clot-derived factors were secreted directly into the original serum volume during conditioning. Therefore, the priority is to maintain a high concentration of the hypoxia-induced pro-angiogenic factors such as VEGF (produced into conditioned medium), while at the same time also delivering platelet-derived factors (released into serum) at naturally occurring concentrations.

Clot conditioned medium can be collected at defined points in time from the start of incubation, e.g. at 1, 2, 3, 4, 5, 6, or 7 days and replaced by new fresh medium, thus allowing the incubation to continue. Through this, different compositions can be obtained that comprise different factor concentrations and ratios, since it is known that hypoxia-induced factor expression by cells varies over time. These compositions then recapitulate the natural sequential expression of the wound healing phases.

It is understood that the coagulation-induced and hypoxia-induced signalling phases generated within a wound are unique/specific to the individual. In order to maintain this specificity when producing compositions, the blood serum and clot used for conditioning are preferably derived from the same subject. Nonetheless, the mixed (cell-free) composition can be applied to the same subject from whom the blood was obtained (autologous composition) or to a different subject (allogeneic composition).

The composition comprising the two phases can be administered to prevent or treat tissue damage by being topically applied onto a wound or injected intradermally/subcutaneously. The composition can be combined with grafts such as skin and fat grafts in order to improve their take, by increasing blood supply to the graft. The composition can also be used in cosmetic applications in order to improve the appearance of skin that has been altered through chronological ageing or photodamage, and also stimulate new hair growth.

When using the kit for preparing serum and/or hypoxia-conditioned medium derived from blood described herein, the invention can be carried out by performing the following methods:

## Method for withdrawing blood and allowing blood sample to clot

1. Sterilize skin and insert intravenous catheter (e.g. Vasuflo®Int) or Butterfly cannula into peripheral vein.

2. Connect the first Syringe* (e.g. Omnifix®, 30 ml) to a three-way valve (e.g. Discofix®) and connect the three-way valve to a first sterile filter (e.g. 0.2 $\mu$m Sterifix®) in a sterile field (*syringe certified for incubation under 37°C for 1-7 days. Syringe may be marked with Up and Down).

3. Connect the first syringe (with attached three-way valve and attached filter) to the catheter or cannula and withdraw blood (ca.10-20 ml).

4. Remove catheter or cannula and place plaster on skin.

5. Disconnect the first syringe from the catheter or cannula, and dispose catheter or cannula in a sharps container. The three-way valve and the first filter remain connected to the first syringe.

6. **While holding the first syringe with blood upright** (opening pointing upwards), draw air (ca. 1-10 ml) through the first filter into the first syringe.

7. Disconnect first filter from three-way valve and discard first filter.

8. While holding first syringe upright place cover (e.g. Combi Stopper) onto the three-way valve, and place first syringe into a holding stand or rack in the upright position.

## Method for obtaining serum and mixing serum into skincare product

1. Following incubation of blood under 37°C for 1-7 days, remove first syringe from incubator, **always holding it in the upright position.**

2. Remove cover from three-way valve and connect second sterile filter (e.g. 0.2 $\mu$m Sterifix®) to the three-way valve.

3. Push plunger of first 30 ml syringe upwards (while holding the first syringe in the upright position) to expel all air through the second filter.

4. Disconnect second filter and discard it.

5. While holding the first syringe in the upright position attach the second, 10 ml Luer-Lock syringe onto 3-way valve and push the plunger of the first, 30 ml syringe so that serum enters the second 10 ml syringe.

6. Collect the complete serum volume, without disturbing the clot in the first 30 ml syringe.

7. Detach the second 10 ml syringe from first 30 ml syringe and safely dispose the first 30 ml syringe.

8. Attach 0.2 $\mu$m filter (i.e. third filter) onto second 10 ml syringe and filter serum while adding it to the skincare product. If the filter gets blocked, replace this with another available filter (i.e. fourth filter).

9. Mix serum into skincare product with spatula.

## Method for obtaining serum and hypoxia-conditioned medium:

### Step 1

1. After taking blood from peripheral vein, allow the blood to clot or centrifuge the blood to induce clotting, while keeping the first syringe* in the upright position**, as described above in section *"Method for withdrawing blood and allowing blood sample to clot"* (*syringe certified for incubation under 37°C for 1-7 days. **Syringe marked with Up and Down indicators: UP corresponds to syringe opening, DOWN corresponds to plunger. Maintain upright position through Steps 1 and 2).

2. Expel air from first syringe as described in paragraphs 1 to 4 of section *"Method for obtaining serum and mixing serum into skincare product"*.

3. Connect a second syringe to the three-way valve. Withdraw the serum into second syringe, without disturbing the clot in the blood-containing syringe (Fig. 1). Disconnect second syringe from three-way valve and store serum-containing second syringe (under low temperature; e.g. frozen between -80°C and -10°C).

4. Place new filter (i.e. a third filter; e.g. 0.2 $\mu$m Sterifix®) onto 3 way valve of blood-containing first syringe and draw air through the third filter into the first syringe. Remove filter from 3-way valve and discard filter. Place cover (e.g. a cap) onto the three-way valve.

5. Connect a third syringe containing sterile medium onto 3 way valve, and add medium to the blood-containing first syringe (Fig. 2). Remove third syringe from three-way valve.

6. Place blood-containing first syringe into a holding stand in the upright position. Incubate the first syringe at 37°C for 1-7 days.

### Step 2

1. Following incubation of blood under 37°C for 1-7 days, remove first syringe from incubator, always holding it in the upright position. Remove cover from three-way valve and connect fourth sterile filter (e.g. 0.2 $\mu$m Sterifix®) to the three-way valve. Push plunger of blood-containing first syringe upwards to expel all air through the fourth filter.

2. Disconnect fourth filter, discard it, and attach an empty fourth syringe onto the 3 way valve.
3. Connect the serum-containing second syringe onto 3 way valve.
4. Collect a defined volume of conditioned medium and serum into the empty fourth syringe to obtain new mixture (Fig. 3).

This new mixture can be filtered using a new 0.2 μm filter (i.e. fifth filter) and can be mixed into a skincare product using a spatula.

Conventionally, blood is not incubated in clinical settings. This is primarily only done in case of blood culture to detect bacterial growth (septic patient). In contrast to this process, here it is important that no contamination of the blood occurs during the incubation phase, since blood-derived growth factors will be used therapeutically. Air must be introduced into the blood-containing syringe because this guarantees blood cell viability, under low oxygen tension (hypoxic) conditions, i.e. avoidance of pathological hypoxia/anoxia (i.e. oxygen tension below 1%, which would reduce factor production by blood cells). In order to ensure that the air introduced into the syringe is sterile, air must be drawn in through the filter. In addition, to avoid growth of pathogens inside the filter and a potential retro-aspiration into the serum or the conditioned medium, the filter should be removed prior to incubation.

Another novel concept is the utilization of the syringe directionally: conventionally, once blood is taken, the way the syringe is positioned is not defined. In the setting of the invention, the first syringe containing blood must always be kept upright (opening up, plunger down) following blot clotting, during the incubation phase and afterwards. The reason for this is to ensure that white blood cells in and around the clot remain in direct contact with the medium, thus getting sufficient nutrients and secreting growth factors into the medium, instead of into the red blood cell layer. Additionally, this prevents mixing of the medium containing the growth factors with red blood cells. Since the white blood cells and clot always end up above the red blood cell layer, having the plunger of the first syringe at the bottom, allows the expulsion of the medium out of the first syringe by pushing the plunger upwards.

The present invention particularly relates to the following 39 items:

1. A kit-of-parts comprising:

(a) a hypoxia-conditioned medium derived from blood; and
(b) a serum

in separate containers.

2. A solution of growth factors comprising a mixture of

(a) a hypoxia-conditioned medium derived from blood; and

(b) a serum.

3. The kit-of parts of item 1 or the solution of item 2, wherein the hypoxia-conditioned medium has a lower concentration of Platelet Factor 4 (PF4) than the serum.

4. The kit-of parts of item 1 or 3 or the solution of item 2 or 3, wherein the hypoxia-conditioned medium contains PF4 in a concentration between 0 ng/ml and 15,000 ng/ml, preferably between 5 ng/ml and 5,000 ng/ml.

5. The kit-of parts of any one of item 1, 3 or 4 or the solution of any one of items 2 to 4, wherein the hypoxia-conditioned medium contains at least one angiogenesis-related growth factor selected from the group consisting of VEGF, TSP1, IL8, Angiogenin (ANG), MMP-9, MMP-8, TIMP-1, and PDGF.

6. The kit-of parts of any one of item 1 or 3 to 5 or the solution of any one of items 2 to 5, wherein the hypoxia-conditioned medium has a higher concentration of Vascular Endothelial Growth Factor (VEGF) than the serum.

7. The kit-of parts of any one of item 1 or 3 to 6 or the solution of any one of items 2 to 6, wherein the VEGF concentration in the hypoxia-conditioned medium is larger than 100 pg/ml.

8. The kit-of parts of any one of item 1 or 3 to 7 or the solution of any one of items 2 to 7, wherein the hypoxia-conditioned medium and/or the serum are derived from one or more blood samples obtained from the same subject.

9. The kit-of parts of any one of item 1 or 3 to 7 or the solution of any one of items 2 to 7, wherein the hypoxia-conditioned medium is derived from one or more blood samples obtained from a first subject and the serum is derived from one or more blood samples obtained from a second subject, wherein the first subject and the second subject are not the same subject.

10. The kit-of parts of any one of item 1 or 3 to 9 or the solution of any one of items 2 to 9, wherein the hypoxia-conditioned medium and/or the serum is free of cells.

11. The solution of any one of items 2 to 10, wherein said solution is free of cells.

12. A method for preparing a kit-of parts, said method comprising the steps:

(i) providing a blood sample from a subject;
(ii) allowing the blood sample of step (i) to clot or inducing clotting in the blood sample of step (i) thereby obtaining a composition comprising a blood clot and serum;
(iii) removing at least a part of the serum from the composition obtained in step (ii) and determining the volume of the serum removed;
(iv) adding medium to the blood clot, wherein the volume of the added medium is equal to or less than the volume of the serum removed in step (iii);

(v) incubating the clot in said medium at a temperature between 10°C and 40°C under hypoxic conditions for 1 to 7 days, thereby obtaining a hypoxia-conditioned medium;

(vi) collecting at least a part of the hypoxia-conditioned medium obtained in step (v); and

(vii) preparing a kit-of-parts comprising:

(a) the hypoxia-conditioned medium collected in step (vi) and

(b) at least a part of the serum removed in step (iii).

13. A method for producing a solution of growth factors, said method comprising the steps:

(i) providing a blood sample from a subject;

(ii) allowing the blood sample of step (i) to clot or inducing clotting in the blood sample of step (i), thereby obtaining a composition comprising a blood clot and serum;

(iii) removing at least a part of the serum from the composition obtained in step (ii) and determining the volume of the serum removed;

(iv) adding medium to the blood clot, wherein the volume of the added medium is equal to or less than the volume of the serum removed in step (iii);

(v) incubating the clot in said medium at a temperature between 10°C and 40°C under hypoxic conditions for 1 to 7 days, thereby obtaining a hypoxia-conditioned medium;

(vi) collecting at least a part of the hypoxia-conditioned medium obtained in step (v);

(vii) mixing the hypoxia-conditioned medium collected in step (vi) with at least a part of the serum removed in step (iii), thereby obtaining the solution of growth factors.

14. A method for preparing a kit-of parts, said method comprising the steps:

(i) providing a first blood sample from a subject;

(ii) allowing the blood sample of step (i) to clot or inducing clotting in the blood sample of step (i), thereby obtaining a first composition comprising a blood clot and serum;

(iii) removing at least a part of the serum from the first composition obtained in step (ii) and determining the volume of the serum removed;

(iv) adding medium to the blood clot, wherein the volume of the added medium is equal to or less than the volume of the serum removed in step (iii);

(v) incubating the clot in said medium at a temperature between 10°C and 40°C under hypoxic conditions for 1 to 7 days, thereby obtaining a hypoxia-conditioned medium;

(vi) collecting at least a part of the hypoxia-conditioned medium obtained in step (v);

(vii) providing a second blood sample from a subject;

(viii) allowing the blood sample of step (vii) to clot or inducing clotting in the blood sample of step (vii), thereby obtaining a second composition comprising a blood clot and serum;

(ix) recovering at least a part of the serum from the second composition obtained in step (viii); and

(x) preparing a kit-of-parts comprising:

(a) the hypoxia-conditioned medium collected in step (vi) and

(b) the serum recovered in step (ix).

15. A method for producing a solution of growth factors, said method comprising the steps:

(i) providing a first blood sample from a subject;

(ii) allowing the blood sample of step (i) to clot or inducing clotting in the blood sample of step (i), thereby obtaining a first composition comprising a blood clot and serum;

(iii) removing at least a part of the serum from the first composition obtained in step (ii) and determining the volume of the serum removed;

(iv) adding medium to the blood clot, wherein the volume of the added medium is equal to or less than the volume of the serum removed in step (iii);

(v) incubating the clot in said medium at a temperature between 10°C and 40°C under hypoxic conditions for 1 to 7 days, thereby obtaining a hypoxia-conditioned medium;

(vi) collecting at least a part of the hypoxia-conditioned medium obtained in step (v);

(vii) providing a second blood sample from a subject;

(viii) allowing the blood sample of step (vii) to clot or inducing clotting in the blood sample of step (vii), thereby obtaining a second composition comprising a blood clot and serum;

(ix) recovering at least a part of the serum from the second composition obtained in step (viii), thereby obtaining a serum; and

(x) mixing the hypoxia-conditioned medium collected in step (vi) with at least a part of the serum recovered in step (ix), thereby obtaining the solution of growth factors.

16. The method of item 14 or item 15, wherein the first blood sample and the second blood sample were obtained from the same subject.

17. The method of any one of items 12 to 16, wherein the hypoxic conditions in the medium in step (v) are achieved through cell-mediated oxygen consumption within a normoxic incubator and/or within an oxygen-regulated incubator.

18. The method of any one of items 12 to 17, wherein the incubation in step (v) is carried out at an oxygen concentration of between 1 and 10%.

19. The method of any one of items 12 to 18, wherein the volume of the medium added in step (iv) is between 1/200 and 2/3 of the volume of the serum removed in step (iii).

20. The method of any one of items 12 to 19, wherein step (vi) comprises the repeated collection of hypoxia-conditioned medium by carrying out the following steps:

(1) collecting at least a part of the hypoxia-conditioned medium at a first time point;
(2) adding medium to the blot clot, thereby replacing the hypoxia-conditioned medium collected in step (1),
(3) continuing incubation,
(4) collecting at least a part of the hypoxia-conditioned medium at a second time point, and
(5) optionally repeating steps (2) to (4) between 1 time and 5 times.

21. The method of item 20, wherein the volume of the medium added in step (iv) and/or the medium added in step (2) is equal to or less than the volume of the serum removed in step (iii), preferably the total volume of the medium added in step (iv) and the medium added in step (2) is equal to or less than the volume of the serum removed in step (iii).

22. The method of any one of items 12 to 21, wherein the solution of growth factors obtained in step (vii) of item 13 or in step (x) of item 15 is obtained by mixing a given volume of hypoxia-conditioned medium ($V_{hcm}$) with a volume of serum ($V_s$) that fulfils the following inequality:

$$V_s \leq \frac{V_{hcm} * V_{S_{total}}}{V_{hcm_{total}}} - V_{hcm}$$

wherein $V_s$ is the volume of serum that can be used for mixing,
wherein $V_{hcm}$ is the volume of hypoxia-conditioned medium used for mixing,
wherein $V_{S_{total}}$ is the total volume of the serum removed in step (iii),
wherein $V_{hcm_{total}}$ is the total volume of the medium added in step (iv), and
wherein $V_{hcm_{total}} \leq V_{S_{total}}$.

23. The method of any one of items 12 to 22, wherein the medium is selected from the group consisting of serum-containing culture medium, serum-free culture medium, a solution that is isotonic with blood, a culture medium with glucose, a culture medium without glucose, a culture medium with albumin and a culture medium without albumin.

24. A kit-of-parts prepared by the method of any one of items 12, 14 or 16 to 20.

25. A solution of growth factors produced by the method of any one of items 13, 15 or 16 to 20.

26. A composition comprising

(a) the solution of any one of items 2 to 11 or 25; and
(b) an excipient.

27. The composition of item 26, wherein the composition is a pharmaceutical composition or a cosmetic composition.

28. The composition of item 26 or 27, wherein the excipient is fibrinogen.

29. The composition of any one of items 26 to 28, wherein the composition further comprises:

(c) a carrier.

30. The composition of item 29, wherein the carrier is in the form of a cream, an emollient, an ointment, a gel, a sol-gel, a spray, a powder, a mesh, a sponge, a patch, a dressing, nanoparticles, microparticles, nanofibers or microfibers.

31. The composition of item 29 or 30, wherein the carrier comprises one or more substances selected from the group consisting of proteins, polysaccharides, glycosaminoglycan, and synthetic polymers.

32. A solution of any one of items 2 to 11 or 25 or a composition of any one of items 26 to 31 for use in medicine.

33. A solution of any one of items 2 to 11 or 25 or a composition of any one of items 26 to 31 for use in the improvement of tissue blood perfusion, for use in the stimulation of angiogenesis, for use in the treatment of skin that has been debrided, for use in the treatment of excessive scarring, for use in the treatment of grafts, for use in the treatment of flaps, for use in the treatment of wounds, and/or for use in the treatment of tissue damage.

34. The solution for use or the composition for use according to item 32 or 33, wherein the solution or the composition is for autologous administration or for allogeneic application.

35. The solution for use or the composition for use according to any one of items 32 to 34, wherein the solution or the composition is for epidermal administration, intradermal administration or for subcutaneous administration.

36. A cosmetic, non-therapeutic use of the solution of any one of items 2 to 11 or 25 or of the composition of any one of items 26 to 31.

37. The cosmetic, non-therapeutic use of item 36, wherein said use is for improving the appearance of skin, for stimulating new hair growth, for augmenting soft tissue, for reducing wrinkles, for skin rejuvenation, for improving the appearance of scars, for reducing excessive skin pigmentation, and/or for improving pigmentation in skin with reduced pigmentation.

38. A kit for preparing serum and/or hypoxia-conditioned medium derived from blood, said kit comprising:

(a) a first syringe,
(b) a second syringe,
(c) optionally a third syringe,
(d) optionally a fourth syringe,
(e) a three-way valve,
(f) a filter,
(g) a cap,
(h) optionally a container containing sterile medium,
(i) optionally a catheter or butterfly cannula,
(j) optionally a sticking plaster,
(k) optionally a rack into which the first syringe can be placed and maintained in upright position, and
(l) optionally a spatula.

39. A use of the kit of item 38 in a method according to any one of items 12 to 23.

**Claims**

1. A kit-of-parts comprising:

(a) a hypoxia-conditioned medium derived from blood; and
(b) a serum

in separate containers.

2. A solution of growth factors comprising a mixture of

(a) a hypoxia-conditioned medium derived from blood; and
(b) a serum.

3. The kit-of parts of claim 1 or the solution of claim 2, wherein

- the hypoxia-conditioned medium has a lower concentration of Platelet Factor 4 (PF4) than the serum, and/or
- the hypoxia-conditioned medium contains PF4 in a concentration between 0 ng/ml and 15,000 ng/ml, preferably between 5 ng/ml and 5,000 ng/ml, and/or
- the hypoxia-conditioned medium contains at least one angiogenesis-related growth factor selected from the group consisting of VEGF, TSP1, IL8, Angiogenin (ANG), MMP-9, MMP-8, TIMP-1, and PDGF, and/or
- the hypoxia-conditioned medium has a higher concentration of Vascular Endothelial Growth Factor (VEGF) than the serum, and/or
- the VEGF concentration in the hypoxia-conditioned medium is greater than 100 pg/ml, and/or
- the hypoxia-conditioned medium and/or the serum are derived from one or more blood samples obtained from the same subject, and/or
- the hypoxia-conditioned medium is derived from one or more blood samples obtained from a first subject and the serum is derived from one or more blood samples obtained from a second subject, wherein the first subject and the second subject are not the same subject, and/or
- the hypoxia-conditioned medium and/or the serum is free of cells.

4. A method for preparing a kit-of parts, said method comprising the steps:

(i) providing a blood sample from a subject;
(ii) allowing the blood sample of step (i) to clot or inducing clotting in the blood sample of step (i) thereby obtaining a composition comprising a blood clot and serum;
(iii) removing at least a part of the serum from the composition obtained in step (ii) and determining the volume of the serum removed;
(iv) adding medium to the blood clot, wherein the volume of the added medium is equal to or less than the volume of the serum removed in step (iii);
(v) incubating the clot in said medium at a temperature between 10°C and 40°C under hypoxic conditions for 1 to 7 days, thereby obtaining a hypoxia-conditioned medium;
(vi) collecting at least a part of the hypoxia-conditioned medium obtained in step (v); and
(vii) preparing a kit-of-parts comprising:

(a) the hypoxia-conditioned medium collected in step (vi) and
(b) at least a part of the serum removed in step (iii).

5. A method for producing a solution of growth factors, said method comprising the steps:

(i) providing a blood sample from a subject;
(ii) allowing the blood sample of step (i) to clot or inducing clotting in the blood sample of step (i), thereby obtaining a composition comprising a blood clot and serum;
(iii) removing at least a part of the serum from the composition obtained in step (ii) and determining the volume of the serum removed;

(iv) adding medium to the blood clot, wherein the volume of the added medium is equal to or less than the volume of the serum removed in step (iii);

(v) incubating the clot in said medium at a temperature between 10°C and 40°C under hypoxic conditions for 1 to 7 days, thereby obtaining a hypoxia-conditioned medium;

(vi) collecting at least a part of the hypoxia-conditioned medium obtained in step (v);

(vii) mixing the hypoxia-conditioned medium collected in step (vi) with at least a part of the serum removed in step (iii), thereby obtaining the solution of growth factors.

6. A method for preparing a kit-of parts, said method comprising the steps:

(i) providing a first blood sample from a subject;

(ii) allowing the blood sample of step (i) to clot or inducing clotting in the blood sample of step (i), thereby obtaining a first composition comprising a blood clot and serum;

(iii) removing at least a part of the serum from the first composition obtained in step (ii) and determining the volume of the serum removed;

(iv) adding medium to the blood clot, wherein the volume of the added medium is equal to or less than the volume of the serum removed in step (iii);

(v) incubating the clot in said medium at a temperature between 10°C and 40°C under hypoxic conditions for 1 to 7 days, thereby obtaining a hypoxia-conditioned medium;

(vi) collecting at least a part of the hypoxia-conditioned medium obtained in step (v);

(vii) providing a second blood sample from a subject;

(viii) allowing the blood sample of step (vii) to clot or inducing clotting in the blood sample of step (vii), thereby obtaining a second composition comprising a blood clot and serum;

(ix) recovering at least a part of the serum from the second composition obtained in step (viii); and

(x) preparing a kit-of-parts comprising:

(a) the hypoxia-conditioned medium collected in step (vi) and
(b) the serum recovered in step (ix).

7. A method for producing a solution of growth factors, said method comprising the steps:

(i) providing a first blood sample from a subject;

(ii) allowing the blood sample of step (i) to clot or inducing clotting in the blood sample of step (i), thereby obtaining a first composition comprising a blood clot and serum;

(iii) removing at least a part of the serum from the first composition obtained in step (ii) and determining the volume of the serum removed;

(iv) adding medium to the blood clot, wherein the volume of the added medium is equal to or less than the volume of the serum removed in step (iii);

(v) incubating the clot in said medium at a temperature between 10°C and 40°C under hypoxic conditions for 1 to 7 days, thereby obtaining a hypoxia-conditioned medium;

(vi) collecting at least a part of the hypoxia-conditioned medium obtained in step (v);

(vii) providing a second blood sample from a subject;

(viii) allowing the blood sample of step (vii) to clot or inducing clotting in the blood sample of step (vii), thereby obtaining a second composition comprising a blood clot and serum;

(ix) recovering at least a part of the serum from the second composition obtained in step (viii), thereby obtaining a serum; and

(x) mixing the hypoxia-conditioned medium collected in step (vi) with at least a part of the serum recovered in step (ix), thereby obtaining the solution of growth factors.

8. A kit-of-parts prepared by the method of any one of claims 4 or 6.

9. A solution of growth factors produced by the method of any one of claims 5 or 7.

10. A composition comprising

(a) the solution of any one of claims 2 to 3 or 9;
(b) an excipient, preferably fibrinogen; and
(c) optionally a carrier.

11. A solution of any one of claims 2 to 3 or 9 or a composition of claim 10 for use in medicine; preferably for use in the improvement of tissue blood perfusion, for use in the stimulation of angiogenesis, for use in the treatment of skin that has been debrided, for use in the treatment of excessive scarring, for use in the treatment of grafts, for use in the treatment of flaps, for use in the treatment of wounds, and/or for use in the treatment of tissue damage.

12. A cosmetic, non-therapeutic use of the solution of any one of claims 2 to 3 or 9 or of the composition of claim 10.

13. The cosmetic, non-therapeutic use of claim 12, wherein said use is for improving the appearance of skin, for stimulating new hair growth, for augmenting soft tissue, for reducing wrinkles, for skin rejuvenation, for improving the appearance of scars, for reducing excessive skin pigmentation, and/or for improving pigmentation in skin with reduced pigmentation.

14. A kit for preparing serum and/or hypoxia-conditioned medium derived from blood, said kit comprising:

(a) a first syringe,
(b) a second syringe,
(c) optionally a third syringe,
(d) optionally a fourth syringe,
(e) a three-way valve,
(f) a filter,
(g) a cap,
(h) optionally a container containing sterile medium,
(i) optionally a catheter or butterfly cannula,
(j) optionally a sticking plaster,
(k) optionally a rack into which the first syringe can be placed and maintained in upright position, and
(l) optionally a spatula.

15. A use of the kit of claim 14 in a method according to any one of claims 4 to 7.

**Fig. 1**

**Fig. 2**

**Fig. 3**

Europäisches
Patentamt

European
Patent Office

Office européen
des brevets

# EUROPEAN SEARCH REPORT

Application Number

EP 16 00 2765

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2015/023911 A1 (SCHILLING ARNDT [DE] ET AL) 22 January 2015 (2015-01-22) | 1-13 | INV. A61K35/14 |
| Y | * paragraphs [0017], [0050], [0056], [0110], [0135] * | 4-7 | |
| X | SUSHMITA ROY ET AL: "Hypoxia improves expansion potential of human cord blood-derived hematopoietic stem cells and marrow repopulation efficiency", EUROPEAN JOURNAL OF HAEMATOLOGY., vol. 88, no. 5, 17 May 2012 (2012-05-17), pages 396-405, XP055349633, DK ISSN: 0902-4441, DOI: 10.1111/j.1600-0609.2012.01759.x * abstract * | 1-3,8-13 | |
| X | EP 2 198 873 A1 (APOSCIENCE AG [AT]) 23 June 2010 (2010-06-23) * abstract * * paragraphs [0029], [0044] * | 1-3,8-13 | TECHNICAL FIELDS SEARCHED (IPC) |
| X | BADER A M ET AL: "Hypoxic preconditioning increases survival and pro-Angiogenic capacity of human cord blood mesenchymal stromal cells in vitro", PLOS ONE 20150918 PUBLIC LIBRARY OF SCIENCE USA, vol. 10, no. 9, 18 September 2015 (2015-09-18), XP002767667, ISSN: 1932-6203 * abstract * * "Cells and cell culture" on 2nd page * | 1-3,8-13 | A61K |

-/--

~~The present search report has been drawn up for all claims~~

| Place of search | Date of completion of the search | Examiner |
|---|---|---|
| Munich | 1 March 2017 | Pilling, Stephen |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt
European
Patent Office
Office européen
des brevets

# EUROPEAN SEARCH REPORT

Application Number

EP 16 00 2765

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | YENG CHIA-HONG ET AL: "Attenuating spinal cord injury by conditioned medium from human umbilical cord blood-derived CD34+cells in rats", TAIWANESE JOURNAL OF OBSTETRICS AND GYNECOLOGY, ELSEVIER (SINGAPORE) PTE LTD, HONG KONG BRANCH, HONG KONG, HK, vol. 55, no. 1, 27 February 2016 (2016-02-27), pages 85-93, XP029435929, ISSN: 1028-4559, DOI: 10.1016/J.TJOG.2015.12.009 * abstract * * "Cell culture and CM" on page 86 * ----- | 1-3,8-13 | |
| X | WO 2014/046417 A1 (MEDIPOST CO LTD [KR]) 27 March 2014 (2014-03-27) * abstract * * page 6, line 31 - page 7, line 15 * * page 8, line 5 - line 10 * ----- | 1-3,8-13 | |
| Y | WO 2014/147092 A1 (KHORIONYX [FR]) 25 September 2014 (2014-09-25) * abstract * * page 5, line 17 - page 6, line 8 * ----- | 4-7 | TECHNICAL FIELDS SEARCHED (IPC) |

~~The present search report has been drawn up for all claims~~

| Place of search | Date of completion of the search | Examiner |
|---|---|---|
| Munich | 1 March 2017 | Pilling, Stephen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-13

☐ The present supplementary European search report has been drawn up for those parts of the European patent application which relate to the invention first mentioned in the claims (Rule 164 (1) EPC).

| | |
|---|---|
| Europäisches Patentamt<br>European Patent Office<br>Office européen des brevets | **LACK OF UNITY OF INVENTION**<br>**SHEET B** |

**Application Number**

EP 16 00 2765

The Search Division considers that the present European patent application does not comply with the requirements of unity of invention and relates to several inventions or groups of inventions, namely:

1. claims: 1-13

   a kit/growth-factor solution comprising a hypoxia-conditioned medium derived from blood and a serum.
   ---

2. claims: 14, 15

   a kit for preparing serum and/or hypoxia-conditioned medium derived from blood, said kit comprising: (a) a first syringe, (b) a second syringe, (c) optionally a third syringe, (d) optionally a fourth syringe, (e) a three-way valve, (f) a filter (g) a cap, (h) optionally a container containing sterile medium, (i) optionally a catheter or butterfly cannula, (j) optionally a sticking plaster, (k) optionally a rack into which the first syringe can be placed and maintained in upright position, and (l) optionally a spatula.
   ---

## ANNEX TO THE EUROPEAN SEARCH REPORT
## ON EUROPEAN PATENT APPLICATION NO.

EP 16 00 2765

This annex lists the patent family members relating to the patent documents cited in the above-mentioned European search report.
The members are as contained in the European Patent Office EDP file on
The European Patent Office is in no way liable for these particulars which are merely given for the purpose of information.

01-03-2017

| Patent document cited in search report | | | Publication date | Patent family member(s) | | | Publication date |
|---|---|---|---|---|---|---|---|
| US 2015023911 | A1 | | 22-01-2015 | AU | 2013214187 | A1 | 10-07-2014 |
| | | | | EP | 2809333 | A1 | 10-12-2014 |
| | | | | US | 2015023911 | A1 | 22-01-2015 |
| | | | | WO | 2013113821 | A1 | 08-08-2013 |
| EP 2198873 | A1 | | 23-06-2010 | AU | 2009336668 | A1 | 07-07-2011 |
| | | | | BR | PI0922174 | A2 | 29-12-2015 |
| | | | | CA | 2746862 | A1 | 15-07-2010 |
| | | | | CN | 102256608 | A | 23-11-2011 |
| | | | | EP | 2198873 | A1 | 23-06-2010 |
| | | | | EP | 2379085 | A1 | 26-10-2011 |
| | | | | ES | 2429518 | T3 | 15-11-2013 |
| | | | | JP | 5869343 | B2 | 24-02-2016 |
| | | | | JP | 2012512842 | A | 07-06-2012 |
| | | | | NZ | 593364 | A | 30-11-2012 |
| | | | | RU | 2011129676 | A | 27-01-2013 |
| | | | | US | 2012045418 | A1 | 23-02-2012 |
| | | | | WO | 2010079086 | A1 | 15-07-2010 |
| | | | | ZA | 201104349 | B | 29-02-2012 |
| WO 2014046417 | A1 | | 27-03-2014 | KR | 20140038236 | A | 28-03-2014 |
| | | | | WO | 2014046417 | A1 | 27-03-2014 |
| WO 2014147092 | A1 | | 25-09-2014 | EP | 2781224 | A1 | 24-09-2014 |
| | | | | EP | 2976098 | A1 | 27-01-2016 |
| | | | | JP | 2016514716 | A | 23-05-2016 |
| | | | | KR | 20150131378 | A | 24-11-2015 |
| | | | | US | 2016279293 | A1 | 29-09-2016 |
| | | | | WO | 2014147092 | A1 | 25-09-2014 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **REINKE JM ; SORG H.** Wound repair and regeneration. *Eur Surg Res,* 2012, vol. 49 (1), 35-43
- A multilingual glossary of biotechnological terms: (IUPAC Recommendations). Helvetica Chimica Acta. 1995
- **CHEEMA, U. ; BROWN, R. A. ; ALP, B. ; MACROBERT, A. J.** Spatially defined oxygen gradients and vascular endothelial growth factor expression in an engineered 3D cell model. *Cellular and Molecular Life Sciences,* 2008, vol. 65, 177-186
- **BHANG, S. H. ; LEE, S. ; SHIN, J. Y. ; LEE, T. J. ; KIM, B. S.** Transplantation of cord blood mesenchymal stem cells as spheroids enhances vascularization. *Tissue Eng Part A,* 2012, vol. 18, 2138-2147